# EUROPEAN PATENT SPECIFICATION

(11) **EP 1 679 946 B1**
(45) Date of publication and mention of the grant of the patent: **30.05.2007**
(21) Application number: 04790939.5
(22) Date of filing: 28.10.2004
(51) Int. Cl.: A01C 1/00, G01J 3/46

(54) **METHOD AND DEVICE FOR DIRECT QUANTITATIVE DETERMINATION OF PESTICIDE SEED LOADING ON INDIVIDUAL SEEDS**
VERFAHREN UND GERÄT FÜR DIE DIREKTE QUANTITATIVE BESTIMMUNG DER PESTIZIDBELASTUNG AN EINZELNEN SAMEN
PROCEDE ET DISPOSITIF DE DETERMINATION QUANTITATIVE DIRECTE DE LA CHARGE DE PESTICIDE SUR DES SEMENCES INDIVIDUELLES

(30) Priority: 29.10.2003 US 515349 P
(43) Date of publication of application: 19.07.2006
(73) Proprietor: Syngenta Participations AG, 4058 Basel (CH)
(72) Inventor: SCHLATTER, Christian, Syngenta Crop Protection AG, CH-4058 Basel (CH); BESTE, Cheryl Lynne, Syngenta Crop Protection, Inc, Greensboro, NC 27409 (US)
(74) Representative: Arunasalam, Velautha-Cumaran
(86) International application number: PCT/EP2004/012166
(87) International publication number: WO 2005/048683

(56) References cited:
- RU-C1- 2 034 295
- US-A- 5 900 944
- US-B1- 6 606 155

## Description

### Field Of The Invention

The invention relates generally to a method and device for pesticide analysis on individual seeds. More particularly to methods and devices for determining quantities of multiple pesticides extracted from seeds of crop plants.

### Background Of The Invention

A reliable determination of pesticide quantities in agricultural and environmental practices is very important. In the agricultural area, pesticides are employed in a variety of ways including coating seeds to protect them against many pests, including being attacked by insects or by soil borne diseases. It is particularly important to control the quantity of pesticides coated on an individual seed because too little pesticide will result in incomplete protection of the seed and emerging plant while too much pesticide may have negative effects on the seed and its germination.

The term "pesticide seed loading", therefore, refers to the quantity of adhered pesticide on the seed.

The current art of quantitative pesticide determination on seeds includes the chromatographic or fluorescence analysis of samples with large numbers of seeds (several grams in weight) or extraction of the dye or pigment from the surface of an individual seed followed by fluorescence analysis. Analysis of larger quantities of seeds shows the average pesticide content of the seeds, however, it does not allow the determination of how well each seed is protected on a single seed basis. The known method of quantification of the dye from a single seed is valid only if the dye content is directly correlated to the pesticide content. This correlation is not always valid, especially in seeds treated with multiple active ingredients having different physico-chemical properties (e.g., melting point, water solubility, particle size). Also, application of this method is obviously limited to seed treatments which include a fluorescent ingredient that can be extracted from the seeds.

One of such methods is known from document US-A-5 900 944.

Current practice in chromatographic analysis of pesticides from coatings of a large sample of seeds cannot be successfully translated to multiple A.I. analysis of single seeds due to several key issues, such as:
- Classical methods of A.I. extraction utilized for gram quantities of seed gave incomplete or erratic results at the trace levels required for single seeds.
- The typical manual solvent addition technique used for extracting the seeds was not practical considering the large number of analyses required for a reliable seed-to-seed distribution determination (typically 60 seeds or more).
- Difficulty is encountered when filtering the extract using a syringe equipped with a syringe filter due to the low volume of extract available and the amount retained on the filter.
- Very dilute extracts of the active ingredients from a single seed provided a significantly sub-optimal matrix for reproducible chromatographic analysis.

The limitations of the art were overcome by the present inventors through a number of experiments which ultimately provided successful results with single seed analysis of commercially important seed treatment active ingredients from crop seeds such as corn, cotton, wheat, soybean and canola.

With the amount of one or more pesticides loading on a seed determined, a plot of the distribution of the seed loading for one or more pesticides for a batch of seeds can be done for each pesticide.

The present invention, therefore, provides an indication of how well seeds are treated with a seed treatment pesticidal composition.

### Summary Of The Invention

According to the present invention, methods and devices are provided for the quantitative analysis of trace levels of multiple pesticides from a single seed.

Direct quantitative determination of multiple active ingredients on a single seed has proved to be a significant challenge due to the extremely low levels of the active ingredients on each individual seed and the difficulty to reproducibly extract these trace quantities. For example, it is not unusual to have loadings as low as 1 µg of each active ingredient on commercially treated seed.

Surprisingly, it has now been found that the methods of high-pressure liquid chromatography or gas chromatography can be reproducibly applied to analysis of multiple pesticides from a single seed by utilization of new seed preparation, extraction, filtration and detection techniques.

Generally, the method comprises the steps of selecting a subset of seeds, extracting the active ingredients from the seed using a suitable extracting fluid based on the active ingredient and seed being extracted (with, for example, by sonication or mechanical shaking), filtration, separation by HPLC or GC, and detection.

The method according to the invention for determining the single seed loading distribution (Gaussian and non-Gaussian) of one or more pesticides on pesticidally treated seeds comprises the steps of:
1) Selecting a subset of seeds sufficient to determine said distribution;
2) Maintaining a seed from said subset in contact with an extracting fluid to substantially selectively extract one or more pesticides from said seed to yield a test sample, and optionally using one or more other extracting fluids to substantially selectively extract one or more other pesticides from the seed to yield one or more other test samples, and optionally then combine the test samples to yield a single test sample;
3) Filtering the test sample containing the pesticide to substantially remove undesired substances extracted from the seed;
4) Separating the one or more pesticides from other substances in the filtered test sample by chromatography;
5) passing the one or more separated pesticides into a detector;
6) Detecting the signal generated by the pesticide at the detector;
7) Relating the amount of signal detected to a quantity of pesticide;
8) Repeating Steps 2-7 sequentially for each seed in said subset;
9) Determining the single seed loading distribution (both Gaussian and non-Gaussian) for the pesticidally treated seeds based on the pesticide quantity determined for each seed in the subset; and
10) Optionally, repeating Steps 4-9 to determine quantity and seed loading distribution of another pesticide in the test sample.

The method is applicable to determination of a broad range of pesticides when loaded on seeds of key agronomic crops such as corn, cotton, wheat, soybean and canola. Suitable crop seeds to be analyzed in accordance with the invention include conventional as well as genetically enhanced or engineered varieties such as, for example, insect resistant (e.g., Bt.) as well as herbicide and disease resistant varieties. Examples of suitable pesticides include azoxystrobin; bitertanol; carboxin; cymoxanil; cyproconazole; cyprodinil; dichlofluamid; difenoconazole; diniconazole; epoxiconazole; fenpiclonil; fludioxonil; fluquiconazole; flusilazole; flutriafol; furalaxyl; guazatin; hexaconazole; hymexazol; imazalil; imibenconazole; ipconazole; kresoxim-methyl; metalaxyl; R-metalaxyl; metconazole; myclobutanil; oxadixyl; pefurazoate; penconazole; pencycuron; prochloraz; propiconazole; pyroquilone; spiroxamin; tebuconazole; thiabendazole; tolifluamide; triazoxide; triadimefon; triadimenol; triflumizole; triticonazole, uniconazole; abamectin; captan; spinosad; emamectin; fipronil; thiacloprid; imidacloprid; thiamethoxam; tefluthrin and chlothianidin. Especially preferred are abamectin; captan; emamectin; fipronil; thiacloprid; imidacloprid; thiamethoxam; tefluthrin; chlothianidin; azoxystrobin; difenoconazole; fludioxonil; flutriafol; ipconazole; metalaxyl; R-metalaxyl; myclobutanil; tebuconazole; and thiabendazole.

### Detailed Description Of The Invention

The present invention enables the reproducible quantitative analysis of trace levels of multiple pesticides from a single seed. The method is workable for pigment-free compositions as well as those containing a pigment (also known as dye or colourant).

### The present invention is described in more detail below:

### Automation Device

Preferably, an automatic solvent addition device that can reproducibly add low volumes of solvent to a large number of samples is required because of the large volume of seeds being analyzed (e.g., 60 for each analysis). The repetitive use of a manual pipette is not preferred due to excessive operator exposure to the solvent and operator errors that can be introduced in such a repetitive and tedious operation. A Hamilton Autodiluter outfitted with a 5 ml syringe was successfully utilized for this purpose, but other devices may also be workable.

### Solvent Choice

A solvent or a combination of solvents (used herein as "extracting fluid") that will reproducibly extract mainly the active ingredients from a single seed is identified. This combination can be different for the different seeds and active ingredients being tested and is not purely based on the solubility of the active ingredients. Generally, a seed is treated with a precise amount of pesticide, and then experiments carried out to determine the extracting fluid that gave the closest to the theoretical amount. For example, thiamethoxam on corn seeds is preferably exposed to water in a pre-wetting or soaking step to effectively remove thiamethoxam from the corn seed. After this wetting step the addition of acetonitrile is used to complete the extraction. When extracting thiamethoxam from cotton seeds a mixture of 50:50 water:acetonitrile can be used in one step with no soaking of the seed. The com extraction scheme (involving soaking of seed in water and then application of acetonitrile) was successfully used with no further testing to remove fludioxonil, mefenoxam, imidacloprid and metalaxyl. However, the addition of acetone was required for extraction of captan from corn seeds, due to its low water solubility.

The composition of the extracting fluid depends on the type of seed, the solubility of the active ingredients applied to the seed and the analytical method that will be used. In general a 50:50 acetonitrile: 0.1% acetic acid mixture is used for HPLC analysis and methyl isobutyl ketone (MIBK) or acetone containing an internal standard such as dimethyl phthalate is used for GC of many common active ingredients off the majority of seeds. Exceptions are many, however, and include corn seeds in general and the active ingredient of captan. One or more extracting fluids may be necessary to effectively extract two or more active ingredients.

In some cases, the extracting fluid may extract from the seed other substances than the pesticides. Examples include compounds that are soluble in the extracting fluid, e.g., colourant, surfactant, emulifiers. These other substances are separated from the pesticides in step 4 using chromatography.

### Filtration Technique

The test sample (the solution after the extraction step) has to be filtered to remove any undesired substances, such as pieces of seed and fibrous material, before analysis by a chromatographic technique. Filtering a small volume (e.g., one milliliter) of the test sample and still obtaining sufficient volume for chromatographic analysis can be difficult. The 13 mm 0.45 micron Acrodisc syringe filters typically used for this purpose retain between 0.5ml and 1.0 ml, leaving an inadequate amount to fill the autosampler vial. A preferred solution involved the use of a Whatmann Uniprep vial which is an autosampler vial with a built-in filter.

### Detection Sources

Types of detectors suitable for use in the present method depend on the chromatographic method used. For example, for HPLC, an UV (ultraviolet) detector, a conductivity detector, a Rl (Refractive index) detector, or a ELS (evaporative light scattering) detector are examples of suitable detectors. Whereas for GC, a FID (flame ionisation detector), an ECD (electron capture detector) or a TCD (thermal conductivity detector) are suitable examples. A UV detector on HPLC and FID on GC have been found to be suitable detectors for a broad range of pesticides.

The mechanism for the generation of the pesticide signal in the detector depends on the type of detector used. For example, in the instance a UV detector is used, the pesticide is illuminated with UV light and the amount of UV light absorbed is measured; in the instance of a conductivity detector is used, the conductivity of the pesticide in the solution is measured; in the instance a RI detector is used, the pesticide is illuminated with beam of radiation light and the resulting refractive index is monitored; and in the instance a FID detector is used, the pesticide is ionized and the resultant increase in ionization current is measured; and in the instance a ECD detector is used, the detector measures the change in standing current due to the capture of electrons by the pesticide, the ECD detector is especially useful for halogenated pesticides.

### Signal Optimisation

Optimizing the signal to allow for detection of the active ingredients, especially when multiple active ingredients were being determined from one seed, is a major hurdle. The test sample could have a concentration about 0.0002 mg/ml because a single seed could be coated with 10 ppm of pesticide and one milliliter of extracting fluid is used. A typical injection volume of 10 microliters into the chromatograph would require detection of amounts as small as 0.2 nanograms of pesticide. Normally when faced with trace level analysis, a chemist can increase the sample size; since this method was not possible for a single seed, the sensitivity can be improved with the following:
- It was found that most pesticide components can be detected at either 265 nm or 230 nm wavelength. Therefore, use of a dual channel detector allows simultaneous detection at both wavelengths thereby optimizing the detection signals.
- Further, an increase of the injection volume to 15 µl led to an in the increase amount of pesticide detected.
- Use of a HPLC column with a smaller internal diameter and smaller particle size (for example, a switch from a column having 150 mm x 4.6 mm diameter, and 5 micron packing to a column with 100 mm x 3.0 mm diameter, and 3 micron packing) was found to increase the sensitivity. Columns of diameter 50 mm x 2.1 mm, 3 micron packing may also be employed in the present invention for highest sensitivity requirements.

Further improvement is possible by optimizing the elution gradient to allow the desired peak (i.e., of the targeted active ingredient) to elute in a clear region of the chromatogram and to shorten elution time to optimize peak shape. This is generally achieved by varying the amount of aqueous eluent to organic eluent.

Technical details for the novel single seed analysis method are provided in the description below.

### Reference solutions

Methods for preparing reference solution are known to a skilled person.

Usually, reference solutions are prepared using reference material (i.e., of the targeted active ingredient) of known purity. In general multiple weights of approximately 0.1 grams is transferred to a 100 ml volumetric flask or a 2 oz. bottle. The weight is recorded from an analytical balance to four decimal places. The volumetric flask is filled to volume with an appropriate solvent (selected based on the solubility of the reference material with the most common solvent being acetonitrile for HPLC analysis and MIBK or acetone containing an internal standard such as dimethyl phthalate for GC analysis). The solution is sonicated or manually shaken until all the reference material is dissolved. This serves as a stock solution.

An appropriate amount of this stock solution is serially diluted using a volumetric pipette into another volumetric flask to prepare the standard solution. For analysis of multiple active ingredients a stock solution for each reference material is made, then all are combined by adding the appropriate amount by volumetric pipette into a fresh volumetric flask to prepare a combined standard solution.

The standard solution is prepared by adding a defined amount of stock solution to a second volumetric flask and then filling it to volume with the extracting fluid used to extract the pesticide from the seed. Again, the solution is sonicated or manually shaken to ensure homogeneity. For consistency with the sample preparation below the solution is preferably filtered using a disposable syringe and filter as its transferred into an autosampler vial. Typically this would be a 0.45 micron Acrodisc filter. The type of filter depends on the solvent used.

### Single seed preparation

A set of seeds is taken from the pesticidally treated seeds that would be representative of them. The number of seeds can vary, but, for statistical purposes, sixty or more are preferred.

Sixty individual seeds, for example, are transferred into separate scintillation vials using care not to disturb the seed coating (in general forceps or a scapula are used). The weight of each seed is recorded using an analytical balance. The weight is recorded to four decimal places.

The suitable extracting fluid is added using, for example, a 5 ml Hamilton autodiluter. The desired amount of extracting fluid is precisely added to each seed. To this end a volumetric pipette operated manually is not recommended. In addition the precision of the autodiluter should be tested prior to its use to ensure it performs adequately.

The amount of the extracting fluid used should be sufficient such that any manipulation (e.g., filtration) of the test sample thereafter would leave enough test sample to carry out the chromatographic analysis.

The volume of extracting fluid added is typically 1-4 ml depending on the size of the seed. The fluid should cover the seed completely to ensure substantial extraction of the pesticide, preferably, 90, more preferably 95, especially 97, %, advantageously complete extraction, yet should be minimized to yield the most concentrated solution. The mixture is preferably sonicated followed if needed by mechanical shaking for a time adequate to substantially extract the active ingredients off the seed and into the extracting fluid and yield the test sample. This time has to be determined experimentally for each combination of seed plus active ingredient by assaying the final solution and evaluating for full recovery of the theoretical amount of each active ingredient. Typically 30 minutes of sonication followed by 15 minutes of mechanical shaking is sufficient time. The test sample is then filtered, for example, into an autosampler vial, preferably using a disposable syringe and a syringe filter (for example, a 0.45 micron Acrodisc filter) unless the total volume of the test sample is 1 ml or less. In that case a Whatmann Uniprep vial, for example, with built-in filter can be used for the autosampler vial to prevent solvent loss within the filter. The filters are chosen based on the fluid being used for extraction.

### Instrumental Analysis

The test sample in the autosampler vials are injected into the instrument using a volume of typically 15 microliters for an HPLC run and 3 microliters for a GC run. An autosampler is preferred because manual injections are not precise enough for the analysis. Separation of the active ingredients is achieved via HPLC or GC.

Suitable HPLC columns include Nucleosil C18, Prism RP, Inertsil ODS-3, Lichrospher NH2, Discovery C18 and many others. The packing material is selected based on the physical properties of the active ingredients being evaluated.

Suitable GC columns include DB-1, DB-5, DB-1701 and many others. Again the column is selected based on the active ingredients being determined.

Appropriate parameters for consideration in HPLC include the length, internal diameter and particle size of the columns. In general a 15 cm or less column is used preferably with 5 micron particles or less and an internal diameter of 4.6 mm or less. In the cases of small seeds or seeds treated with low amounts of active ingredient it is desirable to evaluate the column choice to ensure the smallest peak can be detected.

Similarly for GC it is recommended to use a capillary column and not a wide-bore column to ensure narrow peak shape of the smallest peaks. Detection of the active ingredients is generally done with UV for the HPLC analysis, selected a wavelength optimized for the active ingredients being determined based on their UV response. In general either 265 nm or 230 nm has been shown to be suitable for most active ingredients studied.

### Quantification

Quantification of the amount of each active ingredient is done by measuring the amount of each ingredient seen by the detector, in general by measuring the area of the peak seen. This area is compared to that of the reference solution using in general external calibration for HPLC and internal calibration for GC. An average calibration factor is preferably calculated based on multiple injections of the reference solutions.

The single seed loading distribution of a sub-set of seeds may be carried out on more than one instrument (chromatographic column & detector) so that the results are available faster, for example, a set of 30 seeds on one instrument and another set of 30 seeds on another instrument, and then results consolidated.

### EXAMPLES

Commercial products for seed treatment are mixed with water and optionally a colorant at , laboratory scale according to label instructions. Seed treatment is performed with a Hege 1I Seed Treater (Hege Equipment, Inc., 13915 W. 53rd Street N., Colwich KS). One kilogram of seed are added for each trial. The rotation speed of the Hege is set at 60 rpm. Once the correct rotational speed is achieved, the slurry is added through a syringe over 5 seconds, followed by a 30 second mixing period. Seeds are allowed to air dry for 24 hours prior to analysis.

### Example 1- Analysis of imidacloprid, metalaxyl and captan from corn seeds.

A batch of corn seed is treated with imidacloprid, metalaxyl and captan (applied as Gaucho®, Allegiance® and Captan® 400, respectively) with a target concentration of 500 ppm imidacloprid, 20 ppm metalaxyl, and 460 ppm captan. The samples are analyzed as described below and the resultant seed-to-seed distribution data for this treatment are shown in Tables 1, 2 and 3 for imidacloprid, metalaxyl and captan, respectively.

Eluent preparation (0.1 % acetic acid in water): 1.8 ml of glacial acetic acid is added to 1800 ml of deionized water. Stir well, filter and degas prior to use.

### Part A imidacloprid and metalaxyl standard and sample preparation

Stock preparation for imidacloprid and metalaxyl: Accurately weigh in duplicate (standard A and B) 0.0400- 0.0500 g of imidacloprid primary standard and 0.0300- 0.0400 g of metalaxyl primary standard into separate 2-ounce bottles. Add 50 mL of a 50:50 mixture of acetonitile: deionized water. Sonicate for 30 minutes and mechanically shake for one hour.

Standard preparation for imidacloprid and metalaxyl: Add by pipette 5 mL of imidacloprid stock solution and 5 mL of Metalaxyl stock solution to a 250 mL volumetric flask. Add all A weights to one flask and label A, add all B weights to a separate flask and label B. Fill each flask to volume with 50:50 water: acetonitrile. Invert several times to mix.

Individual corn seed sample preparation for determination of imidacloprid and metalaxyl: Transfer one corn seed into a scintillation vial. Add 2.0 mL of deionized water. Allow to stand undisturbed for 30 minutes. Add 2.0 mL acetonitrile. Sonicate 30 minutes and mechanically shake for one hour. Filter with a 0.45 micron syringe filter prior to analysis.

### Part B Captan standard and sample preparation

Stock preparation: Accurately weight in duplicate (standard A and B) 0.0900- 0.1100 g of captan primary standard into a 2-ounce bottle. Add 50 ml acetone. Sonicate for 30 minutes and mechanically shake for one hour.

Standard preparation for captan: Add by pipette 5 ml of stock solution into a 250 ml volumetric flask. Fill volume with acetone. Invert several times to mix.

Individual corn seed sample preparation for determination of captan: Transfer one corn seed into a scintillation vial. Add 4.0 mL of acetone. Sonicate 30 minutes and mechanically shake for one hour. Filter with a 0.45 micron syringe filter prior to analysis.

### INSTRUMENTATION

Perkin Elmer Series 200 LC pump or equivalent.
Perkin Elmer LC 235 Diode Array detector or equivalent.
Hewlett-Packard Series 1050 autosampler or equivalent
Hamilton MicroLab 1000 autodiluter or equivalent capable of delivering 25.00 ± 0.05 mL aliquots and 2.00 ± 0.05 mL aliquots.
LC Column - Prism RP 150 mm column with 4.6 mm internal diameter and 5 micron particle size. Analytical balance with accuracy of ± 0.1 mg.

### INSTRUMENT CONDITIONS

Detection: UV detection at 265 nm with 5 nm bandwidth and simultaneously UV detection at 230 nm with 5 nm bandwidth
Injection Volume: 10 µl
Flow: 1.0 ml/min
Column Temperature: 35°C
Run Time: Approximately 30 minutes

| Gradient program (linear): | | |
|---|---|---|
| Time [minutes] | 0.1 % acetic acid [%] | acetonitrile [%] |
| 0 | 85 | 15 |
| 5 | 85 | 15 |
| 15 | 25 | 75 |
| 18 | 25 | 75 |
| 21 | 85 | 15 |
| 25 | 85 | 15 |

| Expected Retention Times: | |
|---|---|
| Component | Retention time [Minutes] |
| Imidacloprid | 10.4 |
| | |
| Metalaxyl | 17.1 |
| Captan | 19.5 |

| **TABLE 1** | **IMIDICLOPRID RESULTS** |
|---|---|
| Percent of Averaqe | Number of Seeds |
| 0-4% | - |
| 5-15% | - |
| 16-25% | - |
| 26-35% | - |
| 36-45% | - |
| 46-55% | - |
| 56-65% | 1 |
| 66-75% | 7 |
| 76-85% | 8 |
| 86-95% | 11 |
| 96-105% | 11 |
| 106-115% | 10 |
| 116-125% | 6 |
| 126-135% | 2 |
| 136-145% | 3 |
| 146-155% | 1 |
| 156-165% | - |
| 166-175% | - |
| 176-185% | - |
| 186--195% | - |
| 196-205% | - |
| 206-215% | - |
| 216-225% | - |
| 226-235% | - |
| 236-245% | - |
| 246-250% | - |
| (Average =421 ppm, Target = 500 ppm) | |

| **TABLE 2** | **METALAXYL RESULTS** |
|---|---|
| Percent of Averaqe | Number of Seeds |
| 0-4% | - |
| 5-15% | - |
| 16-25% | 2 |
| 26-35% | 6 |
| 36-45% | 7 |
| 46-55% | 3 |
| 56-65% | 3 |
| 66-75% | 6 |
| 76-85% | 1 |
| 86-95% | 3 |
| 96-105% | 4 |
| 106-115% | 2 |
| 116-125% | 4 |
| 126-135% | 1 |
| 136-145% | 3 |
| 146-155% | 5 |
| 156-165% | 1 |
| 166-175% | 1 |
| 176-185% | 3 |
| 186--195% | - |
| 196-205% | 1 |
| 206-215% | - |
| 216-225% | 1 |
| 226-235% | 2 |
| 236-245% | 1 |
| 246-250% | - |
| (Average = 21 ppm, Target = 20 ppm) | |

| **TABLE 3** | **CAPTAN RESULTS** |
|---|---|
| Percent of Averaqe | Number of Seeds |
| 0-4% | - |
| 5-15% | - |
| 16-25% | - |
| 26-35% | - |
| 36-45% | - |
| 46-55% | - |
| 56-65% | 2 |
| 66-75% | 6 |
| 76-85% | 16 |
| 86-95% | 11 |
| 96-105% | 5 |
| 106-115% | 7 |
| 116-125% | 4 |
| 126-135% | 5 |
| 136-145% | - |
| 146-155% | - |
| 156-165% | 3 |
| 166-175% | - |
| 176-185% | - |
| 186--195% | - |
| 196-205% | - |
| 206-215% | - |
| 216-225% | - |
| 226-235% | - |
| 236-245% | - |
| 246-250% | - |
| (Average = 626 ppm, Target = 460 ppm) | |

### Example 2- Analysis of thiamethoxam, fludioxonil and mefenoxam and myclobutanil from cotton seeds.

A batch of cotton seed is treated with thiamethoxam (applied as Cruiser® 5FS), fludioxonil (applied as Maxim® 4FS), mefenoxam (applied as Apron XL® LS) and myclobutanil (applied as Systhane® WSP) with a target concentration of 3000 ppm, 25 ppm, 75 ppm, and 210 ppm respectively. Tables 4-7 show the seed-to-seed distribution data for this treatment.

Eluent preparation (0.1% acetic acid in water): 1.8 ml of glacial acetic acid is added to 1800 ml of deionized water. Stir well, filter and degas prior to use.

Stock preparation: Accurately weigh in duplicate (standard A and B) 0.0900- 0.1100 g of thiamethoxam primary standard, 0.0450- 0.0550 g of fludioxonil primary standard, 0.0450- 0.0550 g mefenoxam primary standard and 0.0450- 0.0550 g of myclobutanil primary standard into separate 2-ounce bottles. Add 50 mL 50:50 acetonitrile: deionized water. Sonicate for 30 minutes and mechanically shake for one hour.

Standard preparation: Add by pipette 40 mL of thiamethoxam stock solution, 3 mL of mefenoxam stock solution, 1 mL of fludioxonil stock solution and 8 ml myclobutanil stock solution to a 250 mL volumetric flask. Add all A weights to one flask and label A, add all B weights to a separate flask and label B. Fill each flask to volume with 50:50 water: acetonitrile. Invert several times to mix.

Individual cotton seed sample preparation: Transfer one cotton seed into a scintillation vial. Add 3.0 mL of 50:50 acetontrile: deionized water. Sonicate 30 minutes and mechanically shake for one hour. Filter with a 0.45 micron syringe filter prior to analysis.

### INSTRUMENTATION

Perkin Elmer Series 410 LC pump or equivalent.
Perkin Elmer LC 235 Diode Array detector or equivalent.
Hewlett-Packard Series 1050 autosampler or equivalent
Hamilton MicroLab 1000 autodiluter or equivalent capable of delivering 25.00 ± 0.05 mL aliquots and 3.00 ± 0.05 mL aliquots.
LC Column - Prism RP 150 mm column with 4.6 mm internal diameter and 5 micron particle size. Analytical balance with accuracy of ± 0.1 mg.

### INSTRUMENT CONDITIONS

Detection: UV detection at 265 nm with 5 nm bandwidth and simultaneously UV detection at 230 nm with 5 nm bandwidth
Injection Volume: 10 µl
Flow: 1.0 ml/min
Column Temperature:35°C
Run Time: Approximately 30 minutes

| Gradient program (linear): | | |
|---|---|---|
| Time [minutes] | 0.1 % acetic acid [%] | Acetonitrile [%] |
| 0 | 85 | 15 |
| 5 | 85 | 15 |
| 20 | 25 | 75 |
| 23 | 25 | 75 |
| 26 | 85 | 15 |
| 30 | 85 | 15 |

| Expected Retention Times: | |
|---|---|
| Component | Retention time [Minutes] |
| thiamethoxam | 5.9 |
| mefenoxam | 16.6 |
| myclobutanil | 19.4 |
| fludioxonil | 20.2 |

| **TABLE 4** | **Thiamethoxam results** |
|---|---|
| Percent of Averaqe | Number of Seeds |
| 0-4% | - |
| 5-15% | - |
| 16-25% | - |
| 26-35% | - |
| 36-45% | - |
| 46-55% | - |
| 56-65% | 1 |
| 66-75% | 4 |
| 76-85% | 18 |
| 86-95% | 12 |
| 96-105% | 4 |
| 106-115% | 6 |
| 116-125% | 5 |
| 126-135% | 3 |
| 136-145% | 4 |
| 146-155% | 2 |
| 156-165% | - |
| 166-175% | - |
| 176-185% | - |
| 186--195% | - |
| 196-205% | 1 |
| 206-215% | - |
| 216-225% | - |
| 226-235% | - |
| 236-245% | - |
| 246-250% | - |
| (Average = 2457 ppm, Target = 3000 ppm) | |

| **TABLE 5** | **Mefenoxam results** |
|---|---|
| Percent of Average | Number of Seeds |
| 0-4% | - |
| 5-15% | - |
| 16-25% | - |
| 26-35% | - |
| 36-45% | - |
| 46-55% | 2 |
| 56-65% | 8 |
| 66-75% | 10 |
| 76-85% | 13 |
| 86-95% | 7 |
| 96-105% | 2 |
| 106-115% | 3 |
| 116-125% | 6 |
| 126-135% | 3 |
| 136-145% | 2 |
| 146-155% | 1 |
| 156-165% | - |
| 166-175% | - |
| 176-185% | 2 |
| 186--195% | - |
| 196-205% | - |
| 206-215% | - |
| 216-225% | - |
| 226-235% | - |
| 236-245% | - |
| 246-250% | - |
| >250% | 1 |
| (Average = 73 ppm, Target = 75 ppm) | |

| **TABLE 6** | **Fludioxonil results** |
|---|---|
| Percent of Average | Number of Seeds |
| | |
| 0-4% | - |
| 5-15% | - |
| 16-25% | - |
| 26-35% | - |
| 36-45% | - |
| 46-55% | - |
| 56-65% | 1 |
| 66-75% | 9 |
| 76-85% | 12 |
| 86-95% | 11 |
| 96-105% | 7 |
| 106-115% | 3 |
| 116-125% | 7 |
| 126-135% | 4 |
| 136-145% | 4 |
| 146-155% | 1 |
| 156-165% | - |
| 166-175% | - |
| 176-185% | - |
| 186--195% | 1 |
| 196-205% | - |
| 206-215% | - |
| 216-225% | - |
| 226-235% | 2 |
| 236-245% | - |
| 246-250% | - |
| (Average = 21 ppm, Target = 25 ppm) | |

| **TABLE 7** | **Myclobutanil results** |
|---|---|
| Percent of Average | Number of Seeds |
| 0-4% | - |
| 5-15% | - |
| 16-25% | - |
| 26-35% | - |
| 36-45% | - |
| 46-55% | - |
| 56-65% | 1 |
| 66-75% | 7 |
| 76-85% | 15 |
| 86-95% | 12 |
| 96-105% | 4 |
| 106-115% | 7 |
| 116-125% | 2 |
| 126-135% | 5 |
| 136-145% | 4 |
| 146-155% | 2 |
| 156-165% | - |
| 166-175% | - |
| 176-185% | 1 |
| 186-195% | - |
| 196-205% | - |
| 206-215% | - |
| 216-225% | - |
| 226-235% | - |
| 236-245% | - |
| 246-250% | - |
| (Average = 196 ppm, Target = 200 ppm) | |

## Claims

1. A method for determining the single seed loading distribution (Gaussian and non-Gaussian) of one or more pesticides on pesticidally treated seeds comprising the steps of:
1) Selecting a subset of seeds sufficient to determine said distribution;
2) Maintaining a seed from said subset in contact with an extracting fluid to substantially selectively extract one or more pesticides from said seed to yield a test sample, and optionally using one or more other extracting fluids to substantially selectively extract one or more other pesticides from the seed to yield one or more other test samples, and optionally then combine the test samples to yield a single test sample;
3) Filtering the test sample containing the pesticide to substantially remove undesired substances extracted from the seed;
4) Separating the one or more pesticides from other substances in the filtered test sample by chromatography;
5) passing the one or more separated pesticides into a detector;
6) Detecting the signal generated by the pesticide at the detector;
7) Relating the amount of signal detected to a quantity of pesticide;
8) Repeating Steps 2-7 sequentially for each seed in said subset;
9) Determining the single seed loading distribution (both Gaussian and non-Gaussian) for the pesticidally treated seeds based on the pesticide quantity determined for each seed in the subset; and
10) Optionally, repeating Steps 4-9 to determine quantity and seed loading distribution of another pesticide in the test sample.

2. The method according to claim 1 wherein an autodiluter is used in step 2 to add the extracting fluid to the seed.

3. The method according to either claim 1 or claim 2 wherein step 3 is carried out in a autosampler vial with a built-in filter.

4. The method according to any one of claims 1 to 3 wherein a HPLC is used in step 4 and a UV detector having a dual detector of 265nm and 230nm wavelength is used in steps 5 and 6.

5. The method according to claim 4 wherein a HPLC having a column with at most 150mm x 4.6 mm diameter and at most 5 micron packing is used in step 4.

## Patentansprüche

1. Verfahren zum Bestimmen der Verteilung der Beladung (Gauss und Nicht-Gauss) von einem oder mehreren Pestiziden von einzelnem Samen auf pestizidbehandeltem Saatgut, umfassend die Schritte von:
1) Auswählen einer ausreichenden Untermenge von Saatgut, um die Verteilung zu bestimmen;
2) Halten eines Samens aus der Untermenge in Kontakt mit einem Extraktionsfluid, um im Wesentlichen selektiv ein oder mehrere Pestizide aus dem Samen zu extrahieren zur Gewinnung einer Testprobe, und gegebenenfalls Verwenden von einem oder mehreren anderen Extraktionsfluids um im Wesentlichen selektiv ein oder mehrere andere Pestizide aus dem Samen zu extrahieren, zur Gewinnung einer oder mehrerer anderer Testproben, und gegebenenfalls dann Vereinigen der Testproben, um eine einzige Testprobe zu gewinnen;
3) Filtrieren der das Pestizid enthaltenden Testprobe, um aus dem Saatgut extrahierte unerwünschte Substanzen im Wesentlichen zu entfernen;
4) Trennen von einem oder mehreren Pestiziden von anderen Substanzen in der filtrierten Testprobe durch Chromatographie;
5) Leiten von dem einen oder mehreren getrennten Pestiziden zu einem Detektor;
6) Nachweisen des durch das Pestizid an dem Detektor erzeugten Signals;
7) In-Bezug-Setzen des nachgewiesenen Signalbetrags zu einer Menge des Pestizids;
8) Wiederholen von Schritten 2 bis 7 nacheinander für jeden Samen in der Untermenge;
9) Bestimmen der Verteilung der Beladung (Gauss und Nicht-Gauss) für das pestizidbehandelte Saatgut, bezogen auf die für jeden Samen in der Untermenge bestimmte Pestizidmenge; und
10) gegebenenfalls Wiederholen von Schritten 4 bis 9, um die Menge und Verteilung der Beladung von Samen mit weiterem Pestizid in der Testprobe zu bestimmen.

2. Verfahren nach Anspruch 1, wobei ein Verdünnungsautomat in Schritt 2 verwendet wird, um das Extraktionsfluid zu dem Samen zuzusetzen.

3. Verfahren nach einem von Anspruch 1 oder Anspruch 2, wobei Schritt 3 in einem Probennehmerautomaten-Fläschchen mit einem Einbaufilter ausgeführt wird.

4. Verfahren nach einem der Ansprüche 1 bis 3, wobei in Schritt 4 ein HPLC verwendet wird und ein UV-Detektor mit einem Doppeldetektor der Wellenlänge 265 nm und 230 nm in Schritten 5 und 6 verwendet wird.

5. Verfahren nach Anspruch 4, wobei ein HPLC mit einer Säule mit maximal 150 mm, 4,6 mm Durchmesser und einer Füllung von maximal 5 µm in Schritt 4 verwendet wird.

## Revendications

1. Procédé permettant de déterminer la répartition de charge dans une seule semence (distribution gaussienne et non gaussienne) d'un ou plusieurs pesticides dans des semences traitées par pesticide comprenant les étapes consistant à :
1) sélectionner un sous-ensemble de semences suffisant pour déterminer ladite répartition ;
2) maintenir une semence issue dudit sous-ensemble en contact avec un fluide d'extraction pour extraire substantiellement sélectivement un ou plusieurs pesticides de ladite semence afin de produire un échantillon d'analyse, et éventuellement utiliser un ou plusieurs autres fluides d'extraction pour extraire substantiellement sélectivement un ou plusieurs autres pesticides de la semence afin de produire un ou plusieurs autres échantillons d'analyse, et éventuellement combiner ensuite les échantillons d'analyse pour produire un seul échantillon d'analyse ;
3) filtrer l'échantillon d'analyse contenant le pesticide pour retirer sensiblement les substances indésirables extraites de la semence ;
4) séparer par chromatographie le ou les pesticides d'autres substances dans l'échantillon d'analyse filtré ;
5) passer le ou les pesticides séparés dans un détecteur ;
6) détecter le signal généré par le pesticide au niveau du détecteur ;
7) associer la quantité de signal détecté à une quantité de pesticide ;
8) répéter les étapes 2 à 7 par séquence pour chaque semence dudit sous-ensemble ;
9) déterminer la répartition de charge dans une seule semence (distribution gaussienne et non gaussienne) dans les semences traitées par pesticide en se basant sur la quantité de pesticide déterminée pour chaque semence dans le sous-ensemble ; et
10) éventuellement répéter les étapes 4 à 9 pour déterminer la quantité et la répartition de charge dans une semence d'un autre pesticide dans l'échantillon d'analyse.

2. Procédé selon la revendication 1 dans lequel un auto-dilueur est utilisé à l'étape 2 pour ajouter le fluide d'extraction à la semence.

3. Procédé selon la revendication 1 ou 2 dans lequel l'étape 3 est effectuée dans un flacon auto-échantilloneur avec un filtre intégré.

4. Procédé selon l'une quelconque des revendications 1 à 3 dans lequel la technique CLHP est utilisée à l'étape 4, et un détecteur UV ayant un détecteur double à longueurs d'onde de 265 nm et 230 nm est utilisé aux étapes 5 et 6.

5. Procédé selon la revendication 4 dans lequel une technique CLHP ayant une colonne d'un diamètre de 150 mm x 4,6 mm au plus et un remplissage de 5 micromètres au plus est utilisée à l'étape 4.
